# EUROPEAN PATENT APPLICATION

(11) **EP 4 847 608 A1**
(43) Date of publication of application: **02.12.2026**
(21) Application number: 24925359.2
(22) Date of filing: 20.03.2024
(51) Int. Cl.: C12N 9/06, C12N 15/53, C12P 13/00, C12P 41/00

(54) **IMINE REDUCTASE MUTANT AND METHOD FOR PREPARING CHIRAL HYDRAZINE COMPOUND**

(30) Priority: 19.02.2024 CN 202410185492
(71) Applicant: Tianjin Asymchem Biotechnology Co., Ltd., Tianjin 300457 (CN)
(72) Inventor: HONG, Hao, North Carolina 27560 (US); JAMES, Gage, North Carolina 27560 (US); ZHANG, Na, Binhai New Area Tianjin 300457 (CN); JIAO, Xuecheng, Binhai New Area Tianjin 300457 (CN); YANG, Yiming, Binhai New Area Tianjin 300457 (CN); HUANG, Aiping, Binhai New Area Tianjin 300457 (CN); ZHANG, Bifei, Binhai New Area Tianjin 300457 (CN); SHI, Chengcheng, Binhai New Area Tianjin 300457 (CN); MA, Tianjiao, Binhai New Area Tianjin 300457 (CN)
(74) Representative: HGF
(86) International application number: PCT/CN2024/082804
(87) International publication number: WO 2025/175613

(57) **Abstract**

Provided are an imine reductase mutant and a method for preparing a chiral hydrazine compound. The imine reductase mutant includes: (a) a protein with a mutation on the basis of the wild-type imine reductase as shown in SEQ ID NO:1, the mutation including a mutation at position A233; or (b) a protein having 70% or more identity to the amino acid sequence defined in (a) and having imine reductase activity. The present application can solve the problem in the prior art that the activity of a wild-type imine reductase is low, and is suitable for the field of enzyme catalysis.

## Description

This application is based upon and claims the benefit of priority from Chinese Application No. 202410185492.X, filed on 19 February 2024, the application of which is incorporated herein by reference in its entirety.

### Technical Field

The present application relates to the field of enzyme catalysis, and in particular, to an imine reductase mutant and a method for preparing a chiral hydrazine compound.

### Background

Chiral hydrazine is an important structural unit and widely exists in various pharmaceuticals, agricultural chemicals and natural products. The chiral hydrazine compounds can also be used as chiral catalysts in organic synthesis and important intermediates in the synthesis of heterocyclic compounds. Conventional methods for synthesizing chiral hydrazine compounds mainly include a racemate resolution method and an asymmetric synthesis method. Racemate resolution methods pose a certain challenge in separation due to similarities between enantiomers, and suffer from low yields (typically below 50%). Processes for the catalytic reduction of hydrazones to chiral hydrazines using metal catalysts or organic agents typically suffer from expensive metal catalysts or ligands, severe reaction conditions, tedious protection and deprotection steps, low optical purity of the product, etc.

Compared with a chemical synthesis method, a biocatalytic method has many advantages, such as mild reaction conditions, good stereoselectivity of enzymes, and being environmentally friendly, and has been widely applied to industrial production of many pharmaceutical intermediates and fine chemicals. A large amount of imine reductase or reductive amination enzyme is excavated and applied to the reduction of imine, and the reductive amination of ketone or aldehyde to produce an amine compound. However, since the activity of the wild-type imine reductase is poor and the product yield is low, it is difficult to meet the requirements of industrial production.

### Summary

The main object of the present application is to provide an imine reductase mutant and a method for preparing a chiral hydrazine compound, so as to solve the problem in the prior art that the activity of a wild-type imine reductase is low.

In order to achieve the described object, according to a first aspect of the present application, provided is an imine reductase mutant. The imine reductase mutant includes: (a) a protein mutated on the basis of a wild-type imine reductase as shown in SEQ ID NO:1, the mutation including a mutation at position A233; or (b) a protein having 70% or more identity to the amino acid sequence defined in (a) and having imine reductase activity.

Further, the A233 position is mutated to A233C; preferably, the mutation further includes a mutation at any one or more of the following positions: F215, F132, L92, A118, L134, I122, I119, M120, A218, T241, L181, V212, A251, G214, S68, G278, A194, V239, D243, S217, A220, M177, A211, V216 or Y219.

Further, mutations also include mutations at any one or more of the following positions: A220S; L92Y or L92F; A118C or A118I; I122A; I119V; M120F or M120H; F132M, F132L, F132V, F132Q or F132L; L181M, L181I, L181V or L181R; F215M or F215L; A218S, A218I, A218R, A218L, A218V or A218M; V239S, V239M, V239G, V239C, V239I, V239F or V239R; T241C; G278S; A194F, A194K, A194E, A194M, A194V, A194L or A194I; V212L or V212I; A211S, A211R or A211C; A251R, A251G, A251S, A251F or A251C; G214R or G214C; S68A, S68C, S68K or S68T; L134F or L134Y; D243R, D243L, D243V, D243C, D243M or D243G; M177L; V216I or V216L; S217M, S217V, S217L or S217W; Y219V.

Further, the mutations include any one of the following amino acid mutations: A233C; A233C+L92Y; A233C+A118C; A233C+I122A; A233C+I119V; A233C+M120F; A233C+F132M; A233C+F132L; A233C+L181M; A233C+F215M; A233C+F215L; A233C+A218S; A233C+V239S; A233C+T241C; A233C+G278S; A233C+A194F; A233C+F215L+L92F; A233C+F215L+F132L; A233C+F215L+F132M; A233C+F215L+F132V; A233C+F215L+F132Q; A233C+F215L+L181M; A233C+F215L+L181V; A233C+F215L+V212I; A233C+F215L+V239S; A233C+F215L+F132M+L181M; A233C+F215L+F132M+L181V; A233C+F215L+F132M+T241C; A233C+F215L+F132M+A251R; A233C+F215L+F132M+A251G; A233C+F215L+F132M+L92Y; A233C+F215L+F132M+L92F; A233C+F215L+F132M+L92F+A251R; A233C+F215L+F132M+L92F+A251S; A233C+F215L+F132M+L92F+A251F; A233C+F215L+F132M+L92F+A251C; A233C+F215L+F132M+L92F+A251G; A233C+F215L+F132M+L92F+G214R; A233C+F215L+F132M+L92F+S68A; A233C+F215L+F132M+L92F+S68C; A233C+F215L+F132M+L92F+S68K; A233C+F215L+F132M+L92F+G278S; A233C+F215L+F132M+L92F+A118C; A233C+F215L+F132M+L92F+A118I; A233C+F215L+F132M+L92F+M120F; A233C+F215L+F132M+L92F+M120H; A233C+F215L+F132M+L92F+L181V; A233C+F215L+F132M+L92F+L181M; A233C+F215L+F132M+L92F+L134Y; A233C+F215L+F132M+L92F+L134F; A233C+F215L+F132M+L92F+A194K; A233C+F215L+F132M+L92F+A194E; A233C+F215L+F132M+L92F+A118I+V239S; A233C+F215L+F132M+L92F+A118I+V239M; A233C+F215L+F132M+L92F+A118I+V239R; A233C+F215L+F132M+L92F+A118I+D243R; A233C+F215L+F132M+L92F+A118I+D243L; A233C+F215L+F132M+L92F+A118I+L134Y; A233C+F215L+F132M+L92F+A118I+L134F; A233C+F215L+F132M+L92F+A118I+L181M; A233C+F215L+F132M+L92F+A118I+G278S; A233C+F215L+F132M+L92F+A118I+A194M; A233C+F215L+F132M+L92F+A118I+A194V; A233C+F215L+F132M+L92F+A118I+A194L; A233C+F215L+F132M+L92F+A118I+G278S+D243V; A233C+F215L+F132M+L92F+A118I+G278S+D243L; A233C+F215L+F132M+L92F+A118I+G278S+D243R; A233C+F215L+F132M+L92F+A118I+G278S+D243C; A233C+F215L+F132M+L92F+A118I+G278S+L181M; A233C+F215L+F132M+L92F+A118I+G278S+L181R; A233C+F215L+F132M+L92F+A118I+G278S+V239S; A233C+F215L+F132M+L92F+A118I+G278S+A194F; A233C+F215L+F132M+L92F+A118I+G278S+A194M; A233C+F215L+F132M+L92F+A118I+G278S+A194V; A233C+F215L+F132M+L92F+A118I+G278S+A194I; A233C+F215L+F132M+L92F+A118I+G278S+I119V; A233C+F215L+F132M+L92F+A118I+G278S+A194F+S217I; A233C+F215L+F132M+L92F+A118I+G278S+A194F+S217L; A233C+F215L+F132M+L92F+A118I+G278S+A194F+S217V; A233C+F215L+F132M+L92F+A118I+G278S+A194F+A220S; A233C+F215L+F132M+L92F+A118I+G278S+A194F+D243M; A233C+F215L+F132M+L92F+A118I+G278S+A194F+D243G; A233C+F215L+F132M+L92F+A118I+G278S+A194F+D243R; A233C+F215L+F132M+L92F+A118I+G278S+A194F+A218V; A233C+F215L+F132M+L92F+A118I+G278S+A194F+A218I; A233C+F215L+F132M+L92F+A118I+G278S+A194F+A218S; A233C+F215L+F132M+L92F+A118I+G278S+A194F+A218R; A233C+F215L+F132M+L92F+A118I+G278S+A194F+A211C; A233C+F215L+F132M+L92F+A118I+G278S+A194F+A211S; A233C+F215L+F132M+L92F+A118I+G278S+A194F+S68T; A233C+F215L+F132M+L92F+A118I+G278S+A194F+L134F; A233C+F215L+F132M+L92F+A118I+G278S+A194F+L134Y; A233C+F215L+F132M+L92F+A118I+G278S+A194F+L181I; A233C+F215L+F132M+L92F+A118I+G278S+A194F+L181V; A233C+F215L+F132M+L92F+A118I+G278S+A194F+L181M; A233C+F215L+F132M+L92F+A118I+G278S+A194F+M177L; A233C+F215L+F132M+L92F+A118I+G278S+A194F+G214C; A233C+F215L+F132M+L92F+A118I+G278S+A194F+V212L; A233C+F215L+F132M+L92F+A118I+G278S+A194F+V212I; A233C+F215L+F132M+L92F+A118I+G278S+A194F+V239R; A233C+F215L+F132M+L92F+A118I+G278S+A194F+V239G; A233C+F215L+F132M+L92F+A118I+G278S+A194F+V239C; A233C+F215L+F132M+L92F+A118I+G278S+A194F+V239S; A233C+F215L+F132M+L92F+A118I+G278S+A194F+V239I; A233C+F215L+F132M+L92F+A118I+G278S+A194I+V216I; A233C+F215L+F132M+L92F+A118I+G278S+A194I+V216L; A233C+F215L+F132M+L92F+A118I+G278S+A194I+G214C; A233C+F215L+F132M+L92F+A118I+G278S+A194I+S217M; A233C+F215L+F132M+L92F+A118I+G278S+A194I+S217V; A233C+F215L+F132M+L92F+A118I+G278S+A194I+S217L; A233C+F215L+F132M+L92F+A118I+G278S+A194I+S217W; A233C+F215L+F132M+L92F+A118I+G278S+A194I+V212L; A233C+F215L+F132M+L92F+A118I+G278S+A194I+V212I; A233C+F215L+F132M+L92F+A118I+G278S+A194I+A218R; A233C+F215L+F132M+L92F+A118I+G278S+A194I+A218M; A233C+F215L+F132M+L92F+A118I+G278S+A194I+A218L; A233C+F215L+F132M+L92F+A118I+G278S+A194I+S68T; A233C+F215L+F132M+L92F+A118I+G278S+A194I+Y219V; A233C+F215L+F132M+L92F+A118I+G278S+A194I+V239F; A233C+F215L+F132M+L92F+A118I+G278S+A194I+V239I; A233C+F215L+F132M+L92F+A118I+G278S+A194I+A211S; A233C+F215L+F132M+L92F+A118I+G278S+A194I+A211R.

Further, the imine reductase mutant includes a protein having an identity of 75% or more, 80% or more, 85% or more, more preferably 95% or more, further preferably 99% or more to the amino acid sequence defined in (a) and having an imine reductase activity.

In order to achieve the described object, according to a second aspect of the present application, provided is a DNA molecule, the DNA molecule encoding any one of the described imine reductase mutants.

In order to achieve the described object, according to a third aspect of the present application, provided is a recombinant vector, wherein the above DNA molecule is inserted into the recombinant vector.

In order to achieve the described object, according to a fourth aspect of the present application, provided is a host cell, wherein the host cell contains the described DNA molecule or the described recombinant vector.

Further, the host cell includes a prokaryotic cell.

In order to achieve the described object, according to a fourth aspect of the present application, provided is a method for preparing a chiral hydrazine compound. The method includes: catalyzing a reduction reaction between a substrate ketone compound as shown in formula I and a substrate hydrazine compound as shown in formula II using any one of the described imine reductase mutants, so as to obtain a chiral hydrazine compound as shown in formula III;

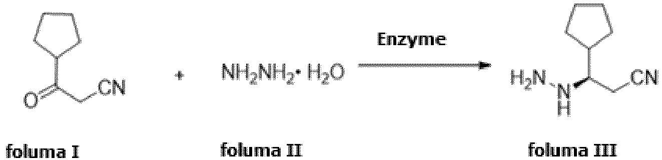

By applying the technical solution of the present application, the described imine reductase mutant is used. Compared with a wild-type enzyme, the described imine reductase mutant has a high enzyme activity, and can greatly reduce the amount of enzyme used in a production process, thereby reducing the production cost, and is suitable for industrial scale-up production.

### Detailed Description of the Embodiments

It is important to note that the embodiments of the present application and the characteristics in the embodiments can be combined under the condition of no conflicts. Hereinafter, the present application will be described in detail with reference to examples.

As mentioned in the background, in the prior art, the activity of a wild-type imine reductase is relatively low, and it is difficult to meet the requirements of industrial production, Therefore, in the present application, the inventors try to develop an imine reductase mutant with a higher enzyme activity, which is used in industrial scale-up production. Based on this, a series of protection solutions of the present application are provided.

In a first typical embodiment of the present application, provided is an imine reductase mutant, including: (a) a protein mutated on the basis of a wild-type imine reductase (named SrIRED in the present application) as shown in SEQ ID NO: 1, the mutation including a mutation at position A233; or (b) a protein having 70% or more identity to the amino acid sequence defined in (a) and having imine reductase activity.

SEQ ID NO:1:





.

In the present application, an enzyme of a wild-type imine reductase having the amino acid sequence (source: Streptosporangium roseum, NCBI Gene Bank: WP_012887675.1) as shown in SEQ ID NO:1 is subjected to an amino acid mutation containing at least an A233 site through enzyme evolution to obtain an imine reductase mutant with an improved activity. SEQ ID NO:2 is a nucleotide sequence encoding the wild type imine reductase.

SEQ ID NO:2:

ATGCGCGATACAGATGTGACCGTTCTGGGTCTGGGTCTGATGGGTCAGGCCCTGGCAGG
TGCATTTCTGAAAGATGGCCATGCCACCACCGTTTGGAATCGCAGCGAAGGCAAAGCAGGTC
AGCTGGCAGAACAGGGTGCAGTGCTGGCAAGTAGCGCCCGTGATGCAGCCGAAGCAAGCCC
TCTGGTGGTGGTTTGCGTGAGTGATCATGCCGCAGTGCGTGCCGTTCTGGATCCGCTGGGTG
ATGTTCTGGCAGGTCGTGTTCTGGTGAACCTGACCAGCGGCACCAGTGAACAGGCACGCGCA
ACAGCAGAATGGGCCGCCGAACGCGGTATCACATACCTGGATGGTGCCATCATGGCCATCCC
GCAGGTGGTTGGTACCGCCGATGCCTTCCTGCTGTATAGCGGCCCGGAAGCCGCCTACGAGG
CACATGAACCTACCCTGCGTAGCCTGGGTGCAGGTACCACATATCTGGGTGCAGATCATGGCC
TGAGCAGCCTGTATGACGTGGCCCTGCTGGGCATCATGTGGGGCACACTGAATAGCTTTCTGC
ACGGCGCAGCCCTGCTGGGCACCGCAAAAGTTGAAGCAACCACCTTTGCCCCGTTTGCCAAC

CGCTGGATCGAAGCCGTTACCGGCTTTGTTAGCGCCTATGCCGGTCAGGTTGATCAGGGTGC
CTATCCGGCACTGGATGCCACCATCGACACCCATGTGGCCACCGTGGATCACCTGATTCATGA
AAGCGAGGCAGCCGGTGTGAATACAGAACTGCCGCGTCTGGTTCGCACCCTGGCCGATCGT
GCACTGGCAGGTGGTCAAGGTGGCCTGGGTTATGCCGCCATGATCGAACAGTTTCGCAGCCC
GAGCGCC.

In a preferred embodiment, in point (a) above, the A233 mutation is A233C; preferably, the mutation further includes a mutation at any one or more of the following positions: F215, F132, L92, A118, L134, I122, I119, M120, A218, T241, L181, V212, A251, G214, S68, G278, A194, V239, D243, S217, A220, M177, A211, V216 or Y219.

In a preferred embodiment, the types of amino acids substituted at each position are each independently selected from the group consisting of:A233C; L92Y or L92F; A118C or A118I; I122A; I119V; M120F or M120H; F132M, F132L, F132V, F132Q or F132L; L181M, L181I, L181V or L181R; F215M or F215L; A218S, A218I, A218R, A218L, A218V or A218M; V239S, V239M, V239G, V239C, V239I, V239F or V239R; T241C; G278S; A194F, A194K, A194E, A194M, A194V, A194L or A194I; V212L or V212I; A211S, A211R or A211C; A251R, A251G, A251S, A251F or A251C; G214R or G214C; S68A, S68C, S68K or S68T; L134F or L134Y; D243R, D243L, D243V, D243C, D243M or D243G; M177L; V216I or V216L; S217M, S217V, S217L or S217W; Y219V; wherein the letter before the number denotes the original amino acid and the letter after the number denotes the mutated amino acid.

In a preferred embodiment, the mutations also include mutations at any one or more of the following positions: A233C; A233C+L92Y; A233C+A118C; A233C+I122A; A233C+I119V; A233C+M120F; A233C+F132M; A233C+F132L; A233C+L181M; A233C+F215M; A233C+F215L; A233C+A218S; A233C+V239S; A233C+T241C; A233C+G278S; A233C+A194F; A233C+F215L+L92F; A233C+F215L+F132L; A233C+F215L+F132M; A233C+F215L+F132V; A233C+F215L+F132Q; A233C+F215L+L181M; A233C+F215L+L181V; A233C+F215L+V212I; A233C+F215L+V239S; A233C+F215L+F132M+L181M; A233C+F215L+F132M+L181V; A233C+F215L+F132M+T241C; A233C+F215L+F132M+A251R; A233C+F215L+F132M+A251G; A233C+F215L+F132M+L92Y; A233C+F215L+F132M+L92F; A233C+F215L+F132M+L92F+A251R; A233C+F215L+F132M+L92F+A251S; A233C+F215L+F132M+L92F+A251F; A233C+F215L+F132M+L92F+A251C; A233C+F215L+F132M+L92F+A251G; A233C+F215L+F132M+L92F+G214R; A233C+F215L+F132M+L92F+S68A; A233C+F215L+F132M+L92F+S68C; A233C+F215L+F132M+L92F+S68K; A233C+F215L+F132M+L92F+G278S; A233C+F215L+F132M+L92F+A118C; A233C+F215L+F132M+L92F+A118I; A233C+F215L+F132M+L92F+M120F; A233C+F215L+F132M+L92F+M120H; A233C+F215L+F132M+L92F+L181V; A233C+F215L+F132M+L92F+L181M; A233C+F215L+F132M+L92F+L134Y; A233C+F215L+F132M+L92F+L134F; A233C+F215L+F132M+L92F+A194K; A233C+F215L+F132M+L92F+A194E; A233C+F215L+F132M+L92F+A118I+V239S; A233C+F215L+F132M+L92F+A118I+V239M; A233C+F215L+F132M+L92F+A118I+V239R; A233C+F215L+F132M+L92F+A118I+D243R; A233C+F215L+F132M+L92F+A118I+D243L; A233C+F215L+F132M+L92F+A118I+L134Y; A233C+F215L+F132M+L92F+A118I+L134F; A233C+F215L+F132M+L92F+A118I+L181M; A233C+F215L+F132M+L92F+A118I+G278S; A233C+F215L+F132M+L92F+A118I+A194M; A233C+F215L+F132M+L92F+A118I+A194V; A233C+F215L+F132M+L92F+A118I+A194L; A233C+F215L+F132M+L92F+A118I+G278S+D243V; A233C+F215L+F132M+L92F+A118I+G278S+D243L; A233C+F215L+F132M+L92F+A118I+G278S+D243R; A233C+F215L+F132M+L92F+A118I+G278S+D243C; A233C+F215L+F132M+L92F+A118I+G278S+L181M; A233C+F215L+F132M+L92F+A118I+G278S+L181R; A233C+F215L+F132M+L92F+A118I+G278S+V239S; A233C+F215L+F132M+L92F+A118I+G278S+A194F; A233C+F215L+F132M+L92F+A118I+G278S+A194M; A233C+F215L+F132M+L92F+A118I+G278S+A194V; A233C+F215L+F132M+L92F+A118I+G278S+A194I; A233C+F215L+F132M+L92F+A118I+G278S+I119V; A233C+F215L+F132M+L92F+A118I+G278S+A194F+S217I; A233C+F215L+F132M+L92F+A118I+G278S+A194F+S217L; A233C+F215L+F132M+L92F+A118I+G278S+A194F+S217V; A233C+F215L+F132M+L92F+A118I+G278S+A194F+A220S; A233C+F215L+F132M+L92F+A118I+G278S+A194F+D243M; A233C+F215L+F132M+L92F+A118I+G278S+A194F+D243G; A233C+F215L+F132M+L92F+A118I+G278S+A194F+D243R; A233C+F215L+F132M+L92F+A118I+G278S+A194F+A218V; A233C+F215L+F132M+L92F+A118I+G278S+A194F+A218I; A233C+F215L+F132M+L92F+A118I+G278S+A194F+A218S; A233C+F215L+F132M+L92F+A118I+G278S+A194F+A218R; A233C+F215L+F132M+L92F+A118I+G278S+A194F+A211C; A233C+F215L+F132M+L92F+A118I+G278S+A194F+A211S; A233C+F215L+F132M+L92F+A118I+G278S+A194F+S68T; A233C+F215L+F132M+L92F+A118I+G278S+A194F+L134F; A233C+F215L+F132M+L92F+A118I+G278S+A194F+L134Y; A233C+F215L+F132M+L92F+A118I+G278S+A194F+L181I; A233C+F215L+F132M+L92F+A118I+G278S+A194F+L181V; A233C+F215L+F132M+L92F+A118I+G278S+A194F+L181M; A233C+F215L+F132M+L92F+A118I+G278S+A194F+M177L; A233C+F215L+F132M+L92F+A118I+G278S+A194F+G214C; A233C+F215L+F132M+L92F+A118I+G278S+A194F+V212L; A233C+F215L+F132M+L92F+A118I+G278S+A194F+V212I; A233C+F215L+F132M+L92F+A118I+G278S+A194F+V239R; A233C+F215L+F132M+L92F+A118I+G278S+A194F+V239G; A233C+F215L+F132M+L92F+A118I+G278S+A194F+V239C; A233C+F215L+F132M+L92F+A118I+G278S+A194F+V239S; A233C+F215L+F132M+L92F+A118I+G278S+A194F+V239I; A233C+F215L+F132M+L92F+A118I+G278S+A194I+V216I; A233C+F215L+F132M+L92F+A118I+G278S+A194I+V216L; A233C+F215L+F132M+L92F+A118I+G278S+A194I+G214C; A233C+F215L+F132M+L92F+A118I+G278S+A194I+S217M; A233C+F215L+F132M+L92F+A118I+G278S+A194I+S217V; A233C+F215L+F132M+L92F+A118I+G278S+A194I+S217L; A233C+F215L+F132M+L92F+A118I+G278S+A194I+S217W; A233C+F215L+F132M+L92F+A118I+G278S+A194I+V212L; A233C+F215L+F132M+L92F+A118I+G278S+A194I+V212I; A233C+F215L+F132M+L92F+A118I+G278S+A194I+A218R; A233C+F215L+F132M+L92F+A118I+G278S+A194I+A218M; A233C+F215L+F132M+L92F+A118I+G278S+A194I+A218L; A233C+F215L+F132M+L92F+A118I+G278S+A194I+S68T; A233C+F215L+F132M+L92F+A118I+G278S+A194I+Y219V; A233C+F215L+F132M+L92F+A118I+G278S+A194I+V239F; A233C+F215L+F132M+L92F+A118I+G278S+A194I+V239I; A233C+F215L+F132M+L92F+A118I+G278S+A194I+A211S; A233C+F215L+F132M+L92F+A118I+G278S+A194I+A211R.

Wherein the letter before the number denotes the original amino acid and the letter after the number denotes the mutated amino acid

Or a protein having 70%, 75%, 80%, 85%, 90%, 95%, 96%, 97%, 98%, 99%, 99.5% or 99.9 percent more identity to the aforementioned amino acid sequences.

In a preferred embodiment, the imine reductase mutant includes a protein having an identity to the amino acid sequence defined in (a) of 75% or more, 80% or more, 85% or more, more preferably 95% or more, further preferably 99% or more, and having imine reductase activity.

The above-mentioned amino acid mutations are all tested and explored in the embodiments of the present application, and compared with the parent having the amino acid sequence as shown in SEQ ID NO:1, all have the activity of reacting the catalytic substrate 3-cyclopentyl-3-oxopropanenitrile with hydrazine hydrate to obtain a chiral hydrazine compound. The above-mentioned mutation sites are all mutations made around amino acid active sites, and such mutations can improve the binding capacity and/or catalytic capacity of a mutant and a substrate. In addition, with regard to a mutation away from an active site, the effect on catalytic capability of an enzyme is relatively small, and therefore, a protein having 70%, 75%, 80%, 85%, 90%, 95%, 96%, 97%, 98%, 99%, 99.5%, or 99.9% or more identity with the described amino acid sequence and having the same catalytic activity can be obtained.

Identity in this description refers to "identity" between amino acid sequences, i.e., the sum of the ratios of amino acid residues in an amino acid sequence that are identical in kind. The identity of amino acid sequences can be determined using BLAST (Basic Local Alignment Search Tool), FASTA, and other alignment programs.

Proteins having 70%, 75%, 80%, 85%, 90%, 95%, and 99% or more (for example, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or more, and even 99.9% or more) identity and having the same function have active sites, active pockets, mechanism of activity, and protein structure that are all the same as those of the protein provided by sequence (a) with a high probability, and are homologous proteins obtained by amino acid mutation.

As used herein, amino acid residues are abbreviated as follows: alanine (Ala; A), Asparagine (Asn; N), aspartic acid (Asp; D), arginine (Arg; R), Cysteine (Cys; C), glutamic acid (Glu; E), Glutamine (Gln; Q), glycine (Gly; G), histidine (His; H), isoleucine (Ile; I), leucine (Leu; L), Lysine (Lys; K), methionine (Met; M), phenylalanine (Phe; F), proline (Pro; P), serine (Ser; S), Threonine (Thr; T), tryptophan (Trp; W), Tyrosine (Tyr; Y) and valine (Val; V).

Rules such as substitution and replacement, in general, the effects after replacement of amino acids with similar properties are also similar. For example, in the homologous proteins described above, conservative amino acid substitutions may occur. "Conservative amino acid substitutions" include, but are not limited to:
Hydrophobic amino acids (Ala, Cys, Gly, Pro, Met, Val, Ile, Leu) are substituted by other hydrophobic amino acids;
The hydrophobic amino acids with a large side chain (Phe, Tyr, Trp) are substituted by other hydrophobic amino acids with a large side chain;
The side chain positively charged amino acids (Arg, His, Lys) are substituted by other side chain positively charged amino acids;
Amino acids with uncharged polar on the side chains (Ser, Thr, Asn, Gln) are substituted by other amino acids with uncharged polar side chains.

A person skilled in the art can also have been able to perform conservative replacements on amino acids according to amino acid replacement rules well known to a person skilled in the art, such as "blosum62 scoring matrix" in the prior art.

The "AlphaFold2-Multimer" used in the present application is a disclosed artificial intelligence model capable of predicting the conformation of a protein complex, and the prediction of the spatial structure of a protein can be very close to the level observed in real tests using equipment such as a cryo-electron microscope. Relatively realistic protein structure can be obtained, thus guiding the investigation of protein structure and protein activity.

In a second exemplary embodiment of the present application, a DNA molecule is provided, the DNA molecule encoding any one of the described imine reductase mutants.

In a third typical embodiment of the present application, a recombinant vector is provided, and the recombinant vector is inserted into the foregoing DNA molecule.

The DNA can encode the imine reductase mutant, and can be connected to a recombinant vector to form a circular DNA. Both the described DNA and recombinant vector can perform transcription and translation under the action of RNA polymerase, ribosome, tRNA, etc., so as to obtain the described imine reductase mutant.

In a fourth typical embodiment of the application, a host cell is provided, which host cell contains the described DNA molecule or recombinant vector.

In a preferred embodiment, the host cell includes a prokaryotic cell; preferably, prokaryotic cells include E. coli; preferably, E. coli includes BL21(DE3). Preferably, the host cell is not a plant cell or animal cell.

The above-mentioned host cell can replicate a recombinant vector in the host cell, and can also perform transcription and translation on a DNA molecule carried on the recombinant vector, so as to obtain a large number of imine reductase mutants. Using whole cells, or by methods known in the art, the host cell is lysed to release proteins, and the resulting crude enzyme preparation is further processed by other means to obtain the imine reductase mutant, and subsequent catalysis on the substrate ketone can be performed. The host cell is a host cell of non-plant or animal origin.

In a fifth exemplary embodiment of the present application, a method for preparing a chiral hydrazine compound is provided, the method includes: providing a method for preparing a chiral hydrazine compound. The method includes: using any one of the imine reductase mutants mentioned above to catalyze a reduction reaction between a substrate ketone compound as shown in formula I and a substrate hydrazine compound as shown in formula II, obtaining a chiral hydrazine compound as shown in formula III;

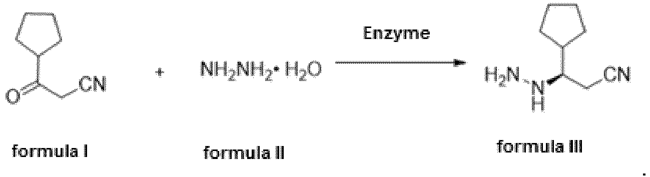

The above-mentioned substrate ketone compound includes 3-cyclopentyl-3-oxopropanenitrile, and the substrate hydrazine compound includes hydrazine hydrate.

Preferably, the reaction temperature of the method is 10°C to 40°C, including but not limited to 10°C, 15°C, 20°C, 25°C, 30°C, 35°C or 40°C; Preferably, the reaction time of the described method is 0.1 - 24 hours, including but not limited to 0.1, 0.2, 0.5, 1, 2, 3, 4, 5, 6, 7, 8, 9, 10, 11, 12, 14, 16, 18, 20, 22 or 24 hours; preferably, the concentration of the substrate ketone compound in the method is 1 µM - 1000 mM, including but not limited to 1 µM, 2 µM, 3 µM, 5 µM, 10 µM, 20 µM, 50 µM, 100 µM, 200 µM, 500 µM, 1 mM, 2 mM, 3 mM, 5 mM, 10 mM, 20 mM, 50 mM, 100 mM, 200 mM, 500 mM or 1000 mM; preferably, the concentration of the substrate hydrazine compound in the described method is 1 µM - 1000 mM, including but not limited to 1 µM, 2 µM, 3 µM, 5 µM, 10 µM, 20 µM, 50 µM, 100 µM, 200 µM, 500 µM, 1 mM, 2 mM, 3 mM, 5 mM, 10 mM, 20 mM, 50 mM, 100 mM, 200 mM, 500 mM or 1000 mM, the concentration of the imine reductase mutant (based on wet-cell crude enzyme solution) is 0.1-1000 mg•mL⁻¹, including but not limited to 0.1, 0.2, 0.3, 0.5, 1, 2, 3, 5, 10, 20, 30, 40, 50, 70, 100, 150, 200, 300, 500, 700, or 1000 mg•mL⁻¹.

Ruxolitinib is a selective JAK1/JAK2 tyrosine kinase inhibitor developed in cooperation by Incyte and Novartis. It is the first drug (trade name: Jakafi) approved by the US FDA in 2011 for treating myelofibrosis, and the drug is indicated for medium-to-high-risk myelofibrosis , including primary myelofibrosis, post-polycythemia vera myelofibrosis, and primary thrombocytosis myelofibrosis, and is marketed as Jakafi.

In the prior art, the chiral hydrazine intermediate is synthesized with a racemate resolution method to further synthesize ruxolitinib, adding and reacting the substrate (E)-3-cyclopentyl acrylonitrile with hydrazine hydrate to produce racemic 3-cyclopentyl-3-hydrazinopropanenitrile, This was then resolved using tartaric acid to give (R)-3-cyclopentyl-3-hydrazinopropanenitrile. However, the above-described racemic resolution method suffers from the problem of low yield. In addition, (R)-3-cyclopentyl-3-hydrazinopropanenitrile can also be prepared by an enzymatic synthesis method, for example, the (R)-3-cyclopentyl-3-hydrazinopropanenitrile can be prepared with an imine reductase SrIRED to catalyze the reaction of a substrate ketone compound 3-cyclopentyl-3-oxopropanenitrile with a substrate hydrazine hydrate; however, since the imine reductase has a poor activity and the product yield is low, the commercial production of an intermediate of ruxolitinib synthesized with a biological method is limited.

In the present application, any one of the described imine reductase mutants is used to react 3-cyclopentyl-3-oxopropanenitrile with hydrazine hydrate in an aqueous phase or a two-phase medium, so as to generate a chiral hydrazine compound ruxolitinib intermediate (R)-3-cyclopentyl-3-hydrazinopropanenitrile. When using the imine reductase mutant in the present application to catalyze the reaction of 3-cyclopentyl-3-oxopropanenitrile with hydrazine hydrate to prepare the ruxolitinib intermediate (R)-3-cyclopentyl-3-hydrazinopropanenitrile, compared with the wild-type imine reductase and reductive amination enzyme, the imine reductase mutant in the present application has a high enzyme activity and a good stereoselectivity, and the product yield is significantly improved.

The beneficial effects of the present application will be further explained in detail below in conjunction with specific embodiments.

### Embodiment 1

1. Taking a wild-type imine reductase SrIRED as a parent, constructing a mutant library with a method of site-specific saturation mutagenesis, and then screening the constructed mutant library using a 96-deep well plate for high-throughput activity to obtain an imine reductase mutant.
   1) Mutant culture: adding 500 µL LB medium to each well of a 96-well plate, inoculating single colonies on an agar plate into a deep-well 96-well plate, and culturing same overnight at 37°C and 700 rpm; 100 µL of a bacterial solution grown overnight is transferred to another 96-well plate containing 700 µL of LB medium at 37°C and 700 rpm, and after culturing for 3 h, the bacterial solution OD600 reaches 0.6 to 0.8, 0.1 mM IPTG solution is added, and induction is carried out overnight at 25°C and 200 rpm for about 16 h; centrifuge at 4000 rpm for 10 min, discard the supernatant, and use the whole cells for the reaction.
   2) High-throughput screening system for 96-well plate: adding 475 µL of a 100 mM Tris-HCl buffer with final concentration of 200 mM hydrazine hydrate, 1 mM NADP⁺, 60 mM glucose monohydrate, 1 mg·mL⁻¹ GDH enzyme (glutamate dehydrogenase) powder, shaking and resuspension to each well of the above obtained 96-well plate containing bacterial pellet, then adding 25 µL of the 4 M substrate 3-cyclopentyl-3-oxopropanenitrile, thermostatted at 20°C, and reacting at 700 rpm for 5 h. After the reaction is finished, 500 µL of acetonitrile is added to the system to stop the reaction, and after centrifugation, the supernatant is taken and analysed by LC-MS or HPLC.
   3) Rescreening of mutants: obtaining mutants with improved activity by the described preliminary screening of mutants. Induction culture in a 2L shake flask (optimal condition for inducing expression: 25°C, induction of 0.1 mM IPTG overnight) is carried out, and after a bacterial pellet is obtained by centrifugation, a crude enzyme solution is obtained by means of ultrasonic cell disruption, and then screening is carried out using the crude enzyme solution. The screening conditions are: 2 mL reaction system, 20 mM of substrate 3-cyclopentyl-3-oxopropanenitrile, 200 mM of hydrazine hydrate, 1 mg·mL⁻¹ of GDH, 1 mM of NADP⁺, 60 mM of glucose monohydrate, 100 mg·mL⁻¹ wet-cell crude enzyme solution, 100 mM of Tris-HCl solution containing 5% DMSO, constant temperature shaker at 20°C, 200 rpm reaction for 5 hours. After the reaction is finished, 2 mL of acetonitrile is added to the system to stop the reaction, and after centrifugation, the supernatant is taken, and detection and analysis are performed by LC-MS or HPLC.
2. Construction of the saturated mutant library employs a full-plasmid PCR amplification method. After obtaining a PCR product, the PCR product is digested with a DpnI enzyme, so that a template in the PCR product is removed, and the PCR product is transformed into E. coli BL21 (DE3).

### Embodiment 2

On the basis of the parent SEQ ID NO:1, performing a saturation mutation at a specific site to construct a mutant library, and then performing high-throughput activity screening on the constructed mutant library using a 96-deep well plate, so as to perform a combination mutation on the obtained mutant with improved activity. After the induction culture of a 2L shake flask is performed on a mutant with an improved initial screening activity, the catalytic activity of the mutant is tested according to the following reaction conditions: 2 mL of a reaction system, 20 mM of substrate 3-cyclopentyl-3-oxopropanenitrile, 200 mM of hydrazine hydrate, 1 mg·mL⁻¹ of GDH, 1 mM of NADP⁺,60 mM glucose monohydrate, 100 mg·mL⁻¹ wet-cell crude enzyme solution, 100 mM Tris-HCl solution containing 5% DMSO, the reaction is carried out at 20°C with a thermostatted shaker at 200 rpm for 5 hrs. After the reaction is finished, 2 mL of acetonitrile is added to the system to stop the reaction, the supernatant is taken after centrifugation, and the yield is analyzed by LC-MS or HPLC. 100 µL of a reaction system, 100 µL of a 1 M NaHCO₃ solution, and 400 µL of 5 mg·mL⁻¹ of Marfey's reagent (CAS: 95713-52-3) are added and left at 50°C for 2 hours, 100 µL of a 1 M HCl solution is added, and the ee value of the reaction is detected by HPLC.

The ee value refers to the Enantiomeric excess. The ee value is used to describe the relative abundance of chiral isomers in a product, ee in the present application = ((R)-3-cyclopentyl-3-hydrazinopropanenitrile-(S)-3-cyclopentyl-3-hydrazinopropanenitrile) ÷ ((R)-3-cyclopentyl-3-hydrazinopropanenitrile + (S)-3-cyclopentyl-3-hydrazinopropanenitrile), the units in the formula can be the proportion of the respective mixture, or the concentration, or the peak area, or other parameters, the units in the formula are unified, that is, the ee value can be obtained through calculation. $Yield = \left(actual yield of product\div theoretical yield of product\right) \times 100 % .$

The detection results are shown in Table 1.

**Table 1**

| Enzyme | Yield% | ee value |
|---|---|---|
| WT | 0.051 | 66 |
| A233C | 0.18 | 90 |
| A233C+L92Y | 0.11 | 90 |
| A233C+A118C | 1.12 | 90 |
| A233C+I122A | 0.18 | 90 |
| A233C+I119V | 0.18 | 90 |
| A233C+M120F | 0.87 | 90 |
| A233C+F132M | 1.36 | 90 |
| A233C+F132L | 0.21 | 90 |
| A233C+L181M | 2.31 | 90 |
| A233C+F215M | 6.5 | 91 |
| A233C+F215L | 10.21 | 92 |
| A233C+A218S | 0.51 | 90 |
| A233C+V239S | 3.23 | 91 |
| A233C+T241C | 0.17 | 91 |
| A233C+G278S | 3.15 | 89 |
| A233C+A194F | 0.45 | 90 |

### Embodiment 3

The optimal mutant A233C+F215L obtained in Embodiment 2 is subjected to the next round of saturation mutation and combination mutation for the parent. After induction culture the 2L shake flask, the primary screening mutant with improved activity is subjected to activity screening according to the following reaction conditions, 2 mL reaction system, 20 mM substrate 3-cyclopentyl-3-oxopropanenitrile, 200 mM hydrazine hydrate, 1 mg·mL⁻¹ GDH, 1 mM NADP⁺, 60 mM glucose monohydrate, 100 mg·mL⁻¹ wet-cell crude enzyme solution, 100 mM Tris-HCl solution with 5% DMSO, the reaction is carried out at 20°C with a thermostatted shaker at 200 rpm for 5 hrs. After the reaction is finished, 2 mL of acetonitrile is added to the system to stop the reaction, the supernatant is taken after centrifugation, and the yield is analyzed by HPLC. The ee value of the reaction is measured after the reaction system is derivatized with Marfey's reagent, and the results are shown in Table 2.

**Table 2**

| Imine reductase mutants | Yield% | ee value |
|---|---|---|
| A233C+F215L | 10.21 | 92 |
| A233C+F215L+L92F | 12.31 | 98 |
| A233C+F215L+F132L | 11.56 | 98 |
| A233C+F215L+F132M | 13.26 | 98 |
| A233C+F215L+F132V | 11.02 | 98 |
| A233C+F215L+F132Q | 8.78 | 98 |
| A233C+F215L+L181M | 12.1 | 98 |
| A233C+F215L+L181V | 11.89 | 98 |
| A233C+F215L+V212I | 7.62 | 98 |
| A233C+F215L+V239S | 12.31 | 98 |
| A233C+F215L+F132M+L181M | 13.65 | 98 |
| A233C+F215L+F132M+L181V | 13.31 | 98 |
| A233C+F215L+F132M+T241C | 14.36 | 98 |
| A233C+F215L+F132M+A251R | 13.63 | 98 |
| A233C+F215L+F132M+A251G | 12.36 | 98 |
| A233C+F215L+F132M+L92Y | 15.37 | 98 |
| A233C+F215L+F132M+L92F | 18.62 | 98 |

### Embodiment 4

The optimal mutant A233C+F215L+F132M+L92F obtained in Embodiment 3 is used as a parent, and the next cycle of saturation mutations and combination mutations are performed, after induction culture the 2L shake flask, the primary screening mutant with improved activity is subjected to activity screening according to the following reaction conditions, 2 mL reaction system, 20 mM substrate 3-cyclopentyl-3-oxopropanenitrile, 200 mM hydrazine hydrate, 1 mg·mL⁻¹ GDH, 1 mM NADP⁺, 60 mM glucose monohydrate, 100 mg·mL⁻¹ wet-cell crude enzyme solution, 100 mM Tris-HCl solution with 5% DMSO, the reaction is carried out at 20°C with a thermostatted shaker at 200 rpm for 5 hrs. After the reaction is finished, 2 mL of acetonitrile is added to the system to stop the reaction, the supernatant is taken after centrifugation, and the yield is analyzed by HPLC. The ee value of the reaction is measured after the reaction system is derivatized with Marfey's reagent, and the results are shown in Table 3.

**Table 3**

| Imine reductase mutants | Yield% | ee value |
|---|---|---|
| A233C+F215L+F132M+L92F | 18.62 | 98 |
| A233C+F215L+F132M+L92F+A251R | 22.01 | 99 |
| A233C+F215L+F132M+L92F+A251S | 21.36 | 99 |
| A233C+F215L+F132M+L92F+A251F | 20.11 | 99 |
| A233C+F215L+F132M+L92F+A251C | 21.12 | 99 |
| A233C+F215L+F132M+L92F+A251G | 21.03 | 99 |
| A233C+F215L+F132M+L92F+G214R | 19.36 | 99 |
| A233C+F215L+F132M+L92F+S68A | 21.63 | 99 |
| A233C+F215L+F132M+L92F+S68C | 21.36 | 99 |
| A233C+F215L+F132M+L92F+S68K | 20.26 | 99 |
| A233C+F215L+F132M+L92F+G278S | 21.78 | 99 |
| A233C+F215L+F132M+L92F+A118C | 21.01 | 99 |
| A233C+F215L+F132M+L92F+A118I | 23.05 | 99 |
| A233C+F215L+F132M+L92F+M120F | 19.01 | 99 |
| A233C+F215L+F132M+L92F+M120H | 18.91 | 99 |
| A233C+F215L+F132M+L92F+L181V | 18.15 | 99 |
| A233C+F215L+F132M+L92F+L181M | 19.26 | 99 |
| A233C+F215L+F132M+L92F+L134Y | 20.32 | 99 |
| A233C+F215L+F132M+L92F+L134F | 20.65 | 99 |
| A233C+F215L+F132M+L92F+A194K | 19.69 | 99 |
| A233C+F215L+F132M+L92F+A194E | 19.23 | 99 |
| A233C+F215L+F132M+L92F+A118I+V239S | 26.36 | 99 |
| A233C+F215L+F132M+L92F+A118I+V239M | 25.13 | 99 |
| A233C+F215L+F132M+L92F+A118I+V239R | 25.11 | 99 |
| A233C+F215L+F132M+L92F+A118I+D243R | 25.32 | 99 |
| A233C+F215L+F132M+L92F+A118I+D243L | 22.36 | 99 |
| A233C+F215L+F132M+L92F+A118I+L134Y | 26.31 | 99 |
| A233C+F215L+F132M+L92F+A118I+L134F | 26.41 | 99 |
| A233C+F215L+F132M+L92F+A118I+L181M | 24.31 | 99 |
| A233C+F215L+F132M+L92F+A118I+A194L | 22.56 | 99 |
| A233C+F215L+F132M+L92F+A118I+G278S | 28.12 | 99 |
| A233C+F215L+F132M+L92F+A118I+A194M | 24.21 | 99 |
| A233C+F215L+F132M+L92F+A118I+A194V | 21.23 | 99 |
| A233C+F215L+F132M+L92F+A118I+A194L | 22.36 | 99 |

### Embodiment 5

The optimal mutant A233C+F215L+F132M+L92F+A118I+G278S obtained in Embodiment 4 is a parent, and the next round of saturation mutation and combination mutation are performed, after the induction culture of the 2L system, mutants with improved initial screening activity are subjected to activity screening according to the following reaction conditions, 2 mL reaction system, 20 mM substrate 3-cyclopentyl-3-oxopropanenitrile, 200 mM hydrazine hydrate, 1 mg·mL⁻¹ GDH, 1 mM NADP⁺, 60 mM glucose monohydrate, 100 mg·mL⁻¹ wet-cell crude enzyme solution, 100 mM Tris-HCl solution with 5% DMSO, the reaction is carried out at 20°C with a thermostatted shaker at 200 rpm for 5 hrs. After the reaction is finished, 2 mL of acetonitrile is added to the system to stop the reaction, the supernatant is taken after centrifugation, and the yield is analyzed by HPLC. The ee value of the reaction is measured after the reaction system is derivatized using Marfey's reagent, and the results are shown in Table 4.

**Table 4**

| Imine reductase mutants | Yield% | ee value |
|---|---|---|
| A233C+F215L+F132M+L92F+A118I+G278S | 28.12 | 99 |
| A233C+F215L+F132M+L92F+A118I+G278S+D243V | 35.36 | 99 |
| A233C+F215L+F132M+L92F+A118I+G278S+D243L | 36.61 | 99 |
| A233C+F215L+F132M+L92F+A118I+G278S+D243R | 34.56 | 99 |
| A233C+F215L+F132M+L92F+A118I+G278S+D243C | 36.25 | 99 |
| A233C+F215L+F132M+L92F+A118I+G278S+L181M | 38.63 | 99 |
| A233C+F215L+F132M+L92F+A118I+G278S+L181R | 34.26 | 99 |
| A233C+F215L+F132M+L92F+A118I+G278S+V239S | 41.69 | 99 |
| A233C+F215L+F132M+L92F+A118I+G278S+A194F | 43.56 | 99 |
| A233C+F215L+F132M+L92F+A118I+G278S+A194M | 38.96 | 99 |
| A233C+F215L+F132M+L92F+A118I+G278S+A194V | 36.65 | 99 |
| A233C+F215L+F132M+L92F+A118I+G278S+A194I | 45.12 | 99 |
| A233C+F215L+F132M+L92F+A118I+G278S+I119V | 31.31 | 99 |
| A233C+F215L+F132M+L92F+A118I+G278S+A194F+A211C | 56.21 | 99 |
| A233C+F215L+F132M+L92F+A118I+G278S+A194F+L181M | 55.36 | 99 |
| A233C+F215L+F132M+L92F+A118I+G278S+A194F+D243M | 59.36 | 99 |
| A233C+F215L+F132M+L92F+A118I+G278S+A194F+D243G | 45.63 | 99 |
| A233C+F215L+F132M+L92F+A118I+G278S+A194F+D243R | 49.36 | 99 |
| A233C+F215L+F132M+L92F+A118I+G278S+A194F+A218V | 50.31 | 99 |
| A233C+F215L+F132M+L92F+A118I+G278S+A194F+A218I | 48.36 | 99 |
| A233C+F215L+F132M+L92F+A118I+G278S+A194F+A218S | 45.36 | 99 |
| A233C+F215L+F132M+L92F+A118I+G278S+A194F+A218R | 39.36 | 99 |
| A233C+F215L+F132M+L92F+A118I+G278S+A194F+A211S | 40.12 | 99 |
| A233C+F215L+F132M+L92F+A118I+G278S+A194F+S68T | 53.23 | 99 |
| A233C+F215L+F132M+L92F+A118I+G278S+A194F+L134F | 69.36 | 99 |
| A233C+F215L+F132M+L92F+A118I+G278S+A194F+L134Y | 65.69 | 99 |
| A233C+F215L+F132M+L92F+A118I+G278S+A194F+L181I | 56.36 | 99 |
| A233C+F215L+F132M+L92F+A118I+G278S+A194F+L181V | 55.32 | 99 |
| A233C+F215L+F132M+L92F+A118I+G278S+A194F+M177L | 68.96 | 99 |
| A233C+F215L+F132M+L92F+A118I+G278S+A194F+G214C | 70.26 | 99 |
| A233C+F215L+F132M+L92F+A118I+G278S+A194F+V212L | 56.32 | 99 |
| A233C+F215L+F132M+L92F+A118I+G278S+A194F+V212I | 65.31 | 99 |
| A233C+F215L+F132M+L92F+A118I+G278S+A194F+V239R | 69.32 | 99 |
| A233C+F215L+F132M+L92F+A118I+G278S+A194F+V239G | 56.31 | 99 |
| A233C+F215L+F132M+L92F+A118I+G278S+A194F+V239C | 55.36 | 99 |
| A233C+F215L+F132M+L92F+A118I+G278S+A194F+V239S | 74.12 | 99 |
| A233C+F215L+F132M+L92F+A118I+G278S+A194F+V239I | 60.31 | 99 |
| A233C+F215L+F132M+L92F+A118I+G278S+A194I+V216I | 55.23 | 99 |
| A233C+F215L+F132M+L92F+A118I+G278S+A194I+V216L | 52.36 | 99 |
| A233C+F215L+F132M+L92F+A118I+G278S+A194I+G214C | 54.36 | 99 |
| A233C+F215L+F132M+L92F+A118I+G278S+A194I+S217M | 56.96 | 99 |
| A233C+F215L+F132M+L92F+A118I+G278S+A194I+S217V | 60.41 | 99 |
| A233C+F215L+F132M+L92F+A118I+G278S+A194I+S217L | 49.26 | 99 |
| A233C+F215L+F132M+L92F+A118I+G278S+A194I+S217W | 54.89 | 99 |
| A233C+F215L+F132M+L92F+A118I+G278S+A194I+V212L | 50.26 | 99 |
| A233C+F215L+F132M+L92F+A118I+G278S+A194I+V212I | 54.21 | 99 |
| A233C+F215L+F132M+L92F+A118I+G278S+A194I+A218R | 53.12 | 99 |
| A233C+F215L+F132M+L92F+A118I+G278S+A194I+A218M | 55.78 | 99 |
| A233C+F215L+F132M+L92F+A118I+G278S+A194I+A218L | 50.46 | 99 |
| A233C+F215L+F132M+L92F+A118I+G278S+A194I+S68T | 57.1 | 99 |
| A233C+F215L+F132M+L92F+A118I+G278S+A194I+Y219V | 59.12 | 99 |
| A233C+F215L+F132M+L92F+A118I+G278S+A194I+V239F | 45.12 | 99 |
| A233C+F215L+F132M+L92F+A118I+G278S+A194I+V239I | 54.71 | 99 |
| A233C+F215L+F132M+L92F+A118I+G278S+A194I+A211S | 61.23 | 99 |
| A233C+F215L+F132M+L92F+A118I+G278S+A194I+A211R | 59.81 | 99 |

From the above description, it can be seen that the above embodiments of the present application achieve the following technical effects: through multiple rounds of evolution, the present application obtains a series of imine reductase mutants with a high activity and good stereoselectivity compared with a wild-type imine reductase; therefore, when the imine reductase mutant obtained in the present application is used to catalytically synthesize a chiral hydrazine compound by reacting 3-cyclopentyl-3-oxopropanenitrile with hydrazine hydrate, the catalytic efficiency is improved, the yield is improved, the stereoselectivity of the reaction is good, and the target product (R)-3-cyclopentyl-3-hydrazinopropanenitrile with a higher purity can be obtained. However, the corresponding isomer (S)-3-cyclopentyl-3-hydrazinopropanenitrile has a low yield and is easy to be purified subsequently. In the technical solution of the present application, the reaction steps of enzymatic synthesis are simple, the usage amount of the enzyme can be greatly reduced, the pollution to the environment caused by the organic solvent is reduced, the production cost is reduced, and the method is environmentally friendly, and the method is suitable for industrial scale-up production.

The above description is only the preferred embodiments of the present application, and is not intended to limit the present application. For those skilled in the art, the present application may have various modifications and variations. Any modifications, equivalent replacements, improvements and the like made within the spirit and principle of the present application shall fall within the protection scope of the present application.

## Claims

1. An imine reductase mutant, wherein the imine reductase mutant comprises:
(a) a protein with a mutation based on a wild-type imine reductase as shown in SEQ ID NO: 1, wherein the mutation comprises a mutation at site A233; or
(b) a protein that has more than 70% identity to the amino acid sequence as shown in (a) and has an imine reductase activity.

2. The imine reductase mutant according to claim 1, wherein the mutation occurring at site A233 is A233C;
preferably, the mutation also comprises a mutation that occurs at any one or more of the following sites: F215, F132, L92, A118, L134, I122, I119, M120, A218, T241, L181, V212, A251, G214, S68, G278, A194, V239, D243, S217, A220, M177, A211, V216 or Y219.

3. The imine reductase mutant according to claim 2, wherein the mutation also comprises a mutation at any one or more of the following sites:
A220S;
L92Y or L92F;
A118C or A118I;
I122A;
I119V;
M120F or M120H;
F132M, F132L, F132V, F132Q or F132L;
L181M, L181I, L181V or L181R;
F215M or F215L;
A218S, A218I, A218R, A218L, A218V or A218M;
V239S, V239M, V239G, V239C, V239I, V239F or V239R;
T241C;
G278S;
A194F, A194K, A194E, A194M, A194V, A194L or A194I;
V212L or V212I;
A211S, A211R or A211C;
A251R, A251G, A251S, A251F or A251C;
G214R or G214C;
S68A, S68C, S68K or S68T;
L134F or L134Y;
D243R, D243L, D243V, D243C, D243M or D243G;
M177L;
V216I or V216L;
S217M, S217V, S217L or S217W;
Y219V.

4. The imine reductase mutant according to claim 1, wherein the mutation comprises any one of the following amino acid mutations:
A233C; A233C+L92Y; A233C+A118C; A233C+I122A; A233C+I119V; A233C+M120F; A233C+F132M; A233C+F132L; A233C+L181M; A233C+F215M; A233C+F215L; A233C+A218S; A233C+V239S; A233C+T241C; A233C+G278S; A233C+A194F; A233C+F215L+L92F; A233C+F215L+F132L; A233C+F215L+F132M; A233C+F215L+F132V; A233C+F215L+F132Q; A233C+F215L+L181M; A233C+F215L+L181V; A233C+F215L+V212I; A233C+F215L+V239S; A233C+F215L+F132M+L181M; A233C+F215L+F132M+L181V; A233C+F215L+F132M+T241C; A233C+F215L+F132M+A251R; A233C+F215L+F132M+A251G; A233C+F215L+F132M+L92Y; A233C+F215L+F132M+L92F; A233C+F215L+F132M+L92F+A251R; A233C+F215L+F132M+L92F+A251S; A233C+F215L+F132M+L92F+A251F; A233C+F215L+F132M+L92F+A251C; A233C+F215L+F132M+L92F+A251G; A233C+F215L+F132M+L92F+G214R; A233C+F215L+F132M+L92F+S68A; A233C+F215L+F132M+L92F+S68C; A233C+F215L+F132M+L92F+S68K; A233C+F215L+F132M+L92F+G278S; A233C+F215L+F132M+L92F+A118C; A233C+F215L+F132M+L92F+A118I; A233C+F215L+F132M+L92F+M120F; A233C+F215L+F132M+L92F+M120H; A233C+F215L+F132M+L92F+L181V; A233C+F215L+F132M+L92F+L181M; A233C+F215L+F132M+L92F+L134Y; A233C+F215L+F132M+L92F+L134F; A233C+F215L+F132M+L92F+A194K; A233C+F215L+F132M+L92F+A194E; A233C+F215L+F132M+L92F+A118I+V239S; A233C+F215L+F132M+L92F+A118I+V239M; A233C+F215L+F132M+L92F+A118I+V239R; A233C+F215L+F132M+L92F+A118I+D243R; A233C+F215L+F132M+L92F+A118I+D243L; A233C+F215L+F132M+L92F+A118I+L134Y; A233C+F215L+F132M+L92F+A118I+L134F; A233C+F215L+F132M+L92F+A118I+L181M; A233C+F215L+F132M+L92F+A118I+G278S; A233C+F215L+F132M+L92F+A118I+A194M; A233C+F215L+F132M+L92F+A118I+A194V; A233C+F215L+F132M+L92F+A118I+A194L; A233C+F215L+F132M+L92F+A118I+G278S+D243V;
A233C+F215L+F132M+L92F+A118I+G278S+D243L;
A233C+F215L+F132M+L92F+A118I+G278S+D243R;
A233C+F215L+F132M+L92F+A118I+G278S+D243C;
A233C+F215L+F132M+L92F+A118I+G278S+L181M;
A233C+F215L+F132M+L92F+A118I+G278S+L181R;
A233C+F215L+F132M+L92F+A118I+G278S+V239S;
A233C+F215L+F132M+L92F+A118I+G278S+A194F;
A233C+F215L+F132M+L92F+A118I+G278S+A194M;
A233C+F215L+F132M+L92F+A118I+G278S+A194V;
A233C+F215L+F132M+L92F+A118I+G278S+A194I;
A233C+F215L+F132M+L92F+A118I+G278S+I119V;
A233C+F215L+F132M+L92F+A118I+G278S+A194F+S217I;
A233C+F215L+F132M+L92F+A118I+G278S+A194F+S217L;
A233C+F215L+F132M+L92F+A118I+G278S+A194F+S217V;
A233C+F215L+F132M+L92F+A118I+G278S+A194F+A220S;
A233C+F215L+F132M+L92F+A118I+G278S+A194F+D243M;
A233C+F215L+F132M+L92F+A118I+G278S+A194F+D243G;
A233C+F215L+F132M+L92F+A118I+G278S+A194F+D243R;
A233C+F215L+F132M+L92F+A118I+G278S+A194F+A218V;
A233C+F215L+F132M+L92F+A118I+G278S+A194F+A218I;
A233C+F215L+F132M+L92F+A118I+G278S+A194F+A218S;
A233C+F215L+F132M+L92F+A118I+G278S+A194F+A218R;
A233C+F215L+F132M+L92F+A118I+G278S+A194F+A211C;
A233C+F215L+F132M+L92F+A118I+G278S+A194F+A211S;
A233C+F215L+F132M+L92F+A118I+G278S+A194F+S68T;
A233C+F215L+F132M+L92F+A118I+G278S+A194F+L134F;
A233C+F215L+F132M+L92F+A118I+G278S+A194F+L134Y;
A233C+F215L+F132M+L92F+A118I+G278S+A194F+L181I;
A233C+F215L+F132M+L92F+A118I+G278S+A194F+L181V;
A233C+F215L+F132M+L92F+A118I+G278S+A194F+L181M;
A233C+F215L+F132M+L92F+A118I+G278S+A194F+M177L;
A233C+F215L+F132M+L92F+A118I+G278S+A194F+G214C;
A233C+F215L+F132M+L92F+A118I+G278S+A194F+V212L;
A233C+F215L+F132M+L92F+A118I+G278S+A194F+V212I;
A233C+F215L+F132M+L92F+A118I+G278S+A194F+V239R;
A233C+F215L+F132M+L92F+A118I+G278S+A194F+V239G;
A233C+F215L+F132M+L92F+A118I+G278S+A194F+V239C;
A233C+F215L+F132M+L92F+A118I+G278S+A194F+V239S;
A233C+F215L+F132M+L92F+A118I+G278S+A194F+V239I;
A233C+F215L+F132M+L92F+A118I+G278S+A194I+V216I;
A233C+F215L+F132M+L92F+A118I+G278S+A194I+V216L;
A233C+F215L+F132M+L92F+A118I+G278S+A194I+G214C;
A233C+F215L+F132M+L92F+A118I+G278S+A194I+S217M;
A233C+F215L+F132M+L92F+A118I+G278S+A194I+S217V;
A233C+F215L+F132M+L92F+A118I+G278S+A194I+S217L;
A233C+F215L+F132M+L92F+A118I+G278S+A194I+S217W;
A233C+F215L+F132M+L92F+A118I+G278S+A194I+V212L;
A233C+F215L+F132M+L92F+A118I+G278S+A194I+V212I;
A233C+F215L+F132M+L92F+A118I+G278S+A194I+A218R;
A233C+F215L+F132M+L92F+A118I+G278S+A194I+A218M;
A233C+F215L+F132M+L92F+A118I+G278S+A194I+A218L;
A233C+F215L+F132M+L92F+A118I+G278S+A194I+S68T;
A233C+F215L+F132M+L92F+A118I+G278S+A194I+Y219V;
A233C+F215L+F132M+L92F+A118I+G278S+A194I+V239F;
A233C+F215L+F132M+L92F+A118I+G278S+A194I+V239I;
A233C+F215L+F132M+L92F+A118I+G278S+A194I+A211S;
A233C+F215L+F132M+L92F+A118I+G278S+A194I+A211R.

5. The imine reductase mutant according to any one of claims 1 to 4, wherein the imine reductase mutant comprises a protein that has more than 75%, more than 80%, more than 85%, more preferably more than 95% and further preferably more than 99% identity to the amino acid sequence as shown in (a) and has the imine reductase activity.

6. A DNA molecule, wherein the DNA molecule encodes the imine reductase mutant according to any one of claims 1 to 5.

7. A recombinant vector, wherein the DNA molecule according to claim 6 is inserted into the recombinant vector.

8. A host cell, wherein the host cell comprises the DNA molecule according to claim 6 or the recombinant vector according to claim 7.

9. The host cell according to claim 8, wherein the host cell comprises prokaryotic cells.

10. A method for preparing a chiral hydrazine compound, wherein the method comprises:
catalyzing a reductive reaction of a substrate ketone compound as shown in formula I and a substrate hydrazine compound as shown in formula II with the imine reductase mutant according to any one of claims 1 to 5,so as to prepare the chiral hydrazine compound as shown in formula III;
